# EUROPEAN PATENT APPLICATION

(11) **EP 4 789 603 A1**
(43) Date of publication of application: **12.08.2026**
(21) Application number: 24874951.7
(22) Date of filing: 02.10.2024
(51) Int. Cl.: A61B 5/00, A61B 5/346, G16H 50/30, G16H 50/70, G16H 10/60, G16H 15/00, G06N 3/045, G06N 3/0464

(54) **DEVICE AND METHOD FOR ESTIMATING BIO-AGE USING BIOMARKER**

(30) Priority: 04.10.2023 KR 20230132027; 19.09.2024 KR 20240126660
(71) Applicant: Seoul National University Hospital, Seoul 03080 (KR)
(72) Inventor: KIM, Joonghee, Seongnam-si, Gyeonggi-do 13620 (KR)
(74) Representative: V.O.
(86) International application number: PCT/KR2024/014984
(87) International publication number: WO 2025/075395

(57) **Abstract**

Disclosed are a device and method for estimating bio-age according to an embodiment. The device for estimating bio-age according to an embodiment comprises: one or more processors; and one or more memories storing instructions executed by the one or more processors. The one or more processors are configured to: extract one or more biomarkers by inputting input data including biological signals of a user to a first model; and estimate the bio-age of the user on the basis of a second model modeled using the one or more biomarkers.

## Description

### [Technical Field]

The disclosed embodiments relate to a technology for estimating bio-age, and more particularly, to a technology for estimating a cardiac bio-age by using a digital biomarker extracted from an electrocardiogram.

### [Cross-Reference to Related Applications]

This application claims priority to Korean Provisional Patent Application No. 10-2023-0132027 filed on October 4, 2023, and Korean Patent Application No. 10-2024-0126660 filed on September 19, 2024, the entire disclosures of which are incorporated herein by reference.

### Background Art

Techniques for directly estimating age from health data including existing electrocardiogram data focus on estimating and learning the actual age of a patient. However, such methods are at significant risk of failing to capture health abnormalities, particularly cardiac health abnormalities, that manifest in various ways.

For example, when the type of disease possessed by a specific person increases or the severity thereof increases, the bio-age should consistently increase. However, since the existing approach takes estimating age as the main training goal, it focuses on learning characteristics related to age rather than characteristics related to diseases.

Therefore, for example, when the heart ages of myocardial infarction patients in their 40s are compared with those of healthy people in their 50s, the age of the 40-year-olds appears to be younger. That is, the existing age estimation methods essentially have an inherent problem that although they may measure age, it is difficult to provide a health-related age indicator, or they may instead output a distorted result.

### [Detailed Description of the Invention]

### [Technical Problem]

Exemplary embodiments of the present invention provide an innovative estimation technique that may estimate a bio-age using a biomarker as intermediate features. Specifically, exemplary embodiments of the present invention provide a technique that may estimate a cardiac bio-age using a biomarker extracted from an electrocardiogram signal as intermediate features.

### [Technical Solution]

A device for estimating a bio-age using a biomarker according to an embodiment includes one or more processors; and one or more memories storing instructions executed by the one or more processors, the one or more processors are configured to: extract one or more biomarkers by inputting input data including biological signals of a user to a pre-trained first model; and estimate a bio-age of the user by inputting the one or more biomarkers to a pre-trained second model.

The biological signal may include an electrocardiogram signal, and the bio-age may include a cardiac bio-age.

The first model may include an artificial intelligence model trained to output one or more corresponding biomarkers when receiving training data related to an electrocardiogram signal, and the second model may include a constrained regression model that models a part of the output data in the training process of the first model as independent variables, is trained to select regression coefficients and independent variables that minimize an error with ground truth values, and is trained to output a corresponding bio-age when receiving values of the output data as input.

The training data of the first model may be configured such that an input value including an electrocardiogram signal; and at least one characteristic among anatomical characteristics, physiological characteristics, and pathological characteristics corresponding to the electrocardiogram signal are labeled as a ground-truth pair.

The anatomical characteristic may be a characteristic related to cardiac hypertrophy, including at least one of the presence or absence of left atrial enlargement (LAE), the presence or absence of left ventricular hypertrophy (LVH), the presence or absence of right atrial enlargement, the presence or absence of right ventricular hypertrophy, a left ventricular mass index, and a left atrial volume index, the physiological characteristic may include at least one of: a characteristic related to systolic function, including the presence or absence of systolic or diastolic dysfunction of a left ventricle or a right ventricle, a left ventricular ejection fraction, myocardial strain, Brain Natriuretic Peptide (BNP), and Pro-brain Natriuretic Peptide (ProBNP); a characteristic related to pulmonary arterial hypertension, including pulmonary artery wedge pressure and the presence or absence of pulmonary hypertension; a characteristic related to cardiac ischemia, including a coronary artery calcium score, the presence or absence of coronary artery stenosis, the presence or absence of cardiac ischemia, and a troponin test result; and a probability of death within a specified period of time, and the pathological characteristic may be a characteristic related to valvular dysfunction, including at least one of the presence or absence or a severity score of stenosis or regurgitation of an aorta, a mitral valve, a lung, or a tricuspid valve.

The one or more processors may be configured to: extract one or more candidate biomarkers by inputting an electrocardiogram signal of the user to the first model; and extract the one or more biomarkers by selecting some of the one or more candidate biomarkers as the one or more biomarkers.

The one or more processors may be configured to: select some of the one or more candidate biomarkers as the one or more biomarkers based on at least one of a correlation between the one or more candidate biomarkers and an actual age of the user, an effect size of the correlation, and a biological plausibility of the one or more candidate biomarkers.

The one or more candidate biomarkers may be a plurality, and the one or more processors may be configured to: cluster the one or more candidate biomarkers into respective groups based on a classification criterion, select one or more representative biomarkers for each of the groups; and estimate the bio-age by inputting the one or more representative biomarkers to the second model.

The classification criterion may include at least one of similarity and biological or medical relevance among the one or more candidate biomarkers.

The one or more processors may be configured to: visually display, on a screen of a device in communication with the processor, the bio-age, together with at least one of: a contribution of the one or more biomarkers in determining the bio-age, a description regarding the contribution, a difference between the user's cardiac bio-age and actual age, a grade corresponding to the difference, a health status according to the grade, and guidance for improving the health status.

The one or more processors may be configured to: perform a linear transformation on the one or more biomarkers such that the one or more biomarkers have a linear correlation having a fixed sign with the user's actual age; and calculate a correlation between the one or more biomarkers that have completed the linear transformation and the actual age.

The second model may include a constrained regression model, and a first constraint of the constrained regression model may be that the one or more representative biomarkers exist in groups, and a second constraint of the constrained regression model may be that the magnitude of the correlation is within a preset range.

The input data may include at least one of a one-dimensional signal representing an electrocardiogram of the user in a time series and a two-dimensional signal representing the electrocardiogram as an image, the first model may include at least one of a vision transformer, a convolutional neural network, and a recurrent neural network, and the one or more processors may be configured to: when a type of the input data is a one-dimensional signal, input the input data into a first-1 model composed of at least one of the convolutional neural network and the recurrent neural network, and when a type of the input data is a two-dimensional signal, input the input data into a first-2 model composed of at least one of the vision transformer and the convolutional neural network.

A method for estimating a bio-age using a biomarker according to an embodiment is performed by a device for estimating a bio-age using a biomarker, including one or more processors; and one or more memories storing instructions executed by one or more processors, where the method includes: extracting one or more biomarkers by inputting input data including biological signals of a user to a pre-trained first model; and estimating a bio-age of the user by inputting the one or more biomarkers to a pre-trained second model.

The biological signal may include an electrocardiogram signal, and the bio-age may include a cardiac bio-age.

The first model may include an artificial intelligence model trained to output one or more corresponding biomarkers when receiving training data related to an electrocardiogram signal, and the second model may include a constrained regression model that models a part of the output data in the training process of the first model as independent variables, is trained to select regression coefficients and independent variables that minimize an error with ground truth values, and is trained to output a corresponding bio-age when receiving values of the output data as input.

The training data of the first model may be configured such that an input value including an electrocardiogram signal; and at least one characteristic among anatomical characteristics, physiological characteristics, and pathological characteristics corresponding to the electrocardiogram signal are labeled as a ground-truth pair.

The anatomical characteristic may be a characteristic related to cardiac hypertrophy, including at least one of the presence or absence of left atrial enlargement (LAE), the presence or absence of left ventricular hypertrophy (LVH), the presence or absence of right atrial enlargement, the presence or absence of right ventricular hypertrophy, a left ventricular mass index, and a left atrial volume index, the physiological characteristic may include at least one of: a characteristic related to systolic function, including the presence or absence of systolic or diastolic dysfunction of a left ventricle or a right ventricle, a left ventricular ejection fraction, myocardial strain, Brain Natriuretic Peptide (BNP), and Pro-brain Natriuretic Peptide (ProBNP); a characteristic related to pulmonary arterial hypertension, including pulmonary artery wedge pressure and the presence or absence of pulmonary hypertension; a characteristic related to cardiac ischemia, including a coronary artery calcium score, the presence or absence of coronary artery stenosis, the presence or absence of cardiac ischemia, and a troponin test result; and a probability of death within a specified period of time, and the pathological characteristic may be a characteristic related to valvular dysfunction, including at least one of the presence or absence and a severity score of stenosis or regurgitation of an aorta, a mitral valve, a lung, or a tricuspid valve.

The step of extracting one or more biomarkers may include: extracting one or more candidate biomarkers by inputting an electrocardiogram signal of the user to the first model; and extracting the one or more biomarkers by selecting some of the one or more candidate biomarkers as the one or more biomarkers.

The step of extracting one or more biomarkers may include selecting some of the one or more candidate biomarkers as the one or more biomarkers based on at least one of a correlation between the one or more candidate biomarkers and an actual age of the user, an effect size of the correlation, and a biological plausibility of the one or more candidate biomarkers.

The one or more candidate biomarkers may be a plurality, and the method may include clustering the one or more candidate biomarkers into respective groups based on a classification criterion, selecting one or more representative biomarkers for each of the groups; and estimating the bio-age by inputting the one or more representative biomarkers to the second model.

The classification criterion may include at least one of similarity and biological or medical relevance among the one or more candidate biomarkers.

The method may include visually displaying, on a screen of the device, the bio-age, together with at least one of: a contribution of the one or more biomarkers in determining the bio-age, a description regarding the contribution, a difference between the user's cardiac bio-age and actual age, a grade corresponding to the difference, a health status according to the grade, and guidance for improving the health status.

The method may further include: performing a linear transformation on the one or more biomarkers such that the one or more biomarkers have a linear correlation having a fixed sign with the user's actual age; and calculating a correlation between the one or more biomarkers that have completed the linear transformation and the actual age.

The second model may include a constrained regression model, a first constraint of the constrained regression model may be that the one or more representative biomarkers exist, and a second constraint of the constrained regression model may be that the magnitude of the correlation is within a preset range.

The input data may include at least one of a one-dimensional signal representing an electrocardiogram of the user in a time series and a two-dimensional signal representing the electrocardiogram as an image, the first model may include at least one of a vision transformer, a convolutional neural network, and a recurrent neural network, and the method may include: when a type of the input data is a one-dimensional signal, inputting the input data into a first-1 model composed of at least one of the convolutional neural network and the recurrent neural network, and when a type of the input data is a two-dimensional signal, inputting the input data into a first-2 model composed of at least one of the vision transformer and the convolutional neural network.

### [Advantageous Effects]

The disclosed embodiments may extract digital biomarkers from an electrocardiogram as intermediate mediators and utilize them to estimate a bio-age that meaningfully reflects cardiac health status, such as the type and severity of cardiac disease burden of a particular individual. Specifically, the disclosed embodiments may extract digital biomarkers capable of estimating anatomical, functional, and pathological characteristics of each individual from the electrocardiogram, transform the extracted digital biomarkers to have a linear and consistent positive correlation with the bio-age of the individual's heart, and additionally estimate a bio-age reflecting health status by combining the biomarkers into a linear regression equation in which respective regression coefficients are constrained within a predetermined range.

The disclosed embodiments estimate the bio-age using a regression model modeled with extracted biomarkers as variables, thereby enhancing interpretability and transparency regarding which biomarkers influence bio-age.

The disclosed embodiments may provide insights for an individual into the interpretation of an estimated bio-age by visually presenting the contribution of each digital biomarker when estimating the bio-age of the individual. That is, the embodiments may provide information on what specific improvements need to be made and what actions may be taken to reduce an individual's estimated bio-age. Through this, personalized and customized medical solutions may be provided.

The disclosed embodiments may associate a difference between an estimated bio-age and an actual age (defined as delta age) with health risks such as risk of death, risk of comorbidities, etc., to provide clinicians with immediate and actionable clinical information for treatment planning.

### [Description of Drawings]

FIG. 1 is a block diagram for describing a device for estimating a bio-age according to an embodiment.
FIG. 2 is a block diagram of a processor included in the device for estimating a bio-age according to an embodiment.
FIG. 3 is an exemplary diagram for describing an operation of the device for estimating a bio-age according to an embodiment.
FIG. 4 is a flowchart for describing a method of estimating a bio-age according to an embodiment.

### [Description of Embodiments]

The foregoing description is provided to assist in a comprehensive understanding of the methods, devices, and/or systems described herein. However, it is merely exemplary and the present disclosure is not limited thereto.

In describing embodiments, if it is determined that a detailed description of known technology related to the present invention may unnecessarily obscure the gist of the embodiment, such detailed description will be omitted.

Terms used below are defined based on functions in the present disclosure, which may vary depending on the intentions or common practices of a user or operator. Therefore, definitions thereof should be made based on the contents throughout the specification. Terms used in the detailed description are merely for describing exemplary embodiments and should not be construed as limiting.

Unless clearly otherwise indicated, the singular forms are intended to include the plural forms. The terms "first," "second," and the like are used merely to distinguish one component from another, and do not limit the components.

Meanwhile, the device of the present disclosure may be entirely hardware-based or may have an aspect that is partially hardware-based and partially software-based. For example, the elderly cognitive impairment severity estimation system and each unit included therein in this specification may generally refer to a device for exchanging data in a specific format and content through an electronic communication manner and software related thereto. Terms such as "unit," "module," "server," "system," "device," and "terminal" in this specification are intended to refer to a combination of hardware and software driven by the hardware. For example, the hardware here may be a data processing device including a CPU or another processor. In addition, software driven by hardware may refer to a running process, an object, an executable, a thread of execution, a program, and the like.

In the present specification, the term "bio-age" refers to an age reflecting a health status of an individual's heart, such as the type and severity of the disease burden, and is a concept different from actual age. For example, when the cardiac bio-age of a 40-year-old myocardial infarction patient with a heart disease is compared with that of a healthy 50-year-old person, the age of the 40-year-old person may be lower. This is different from the actual age of the 50-year-old person and is an age reflecting a condition related to cardiac health.

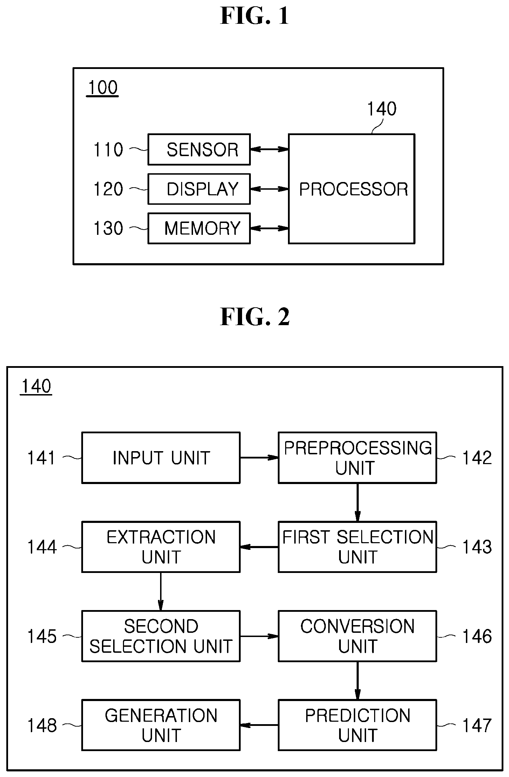

FIG. 1 is a block diagram for describing a device 100 for estimating a bio-age using a biomarker according to an embodiment.

Referring to FIG. 1, the device 100 for estimating a bio-age using a biomarker according to an embodiment includes a sensor 110, a display 120, a memory 130, and a processor 140.

The sensor 110 may sense a health status of a user in contact with the device 100 for estimating a bio-age using a biomarker, and generate an electrical signal and/or a data value corresponding to the sensed status. In particular, the sensor 110 may measure an electrical and physical condition of a heart to generate an electrical signal and/or data processable by the processor 140.

For example, the sensor 110 may include a biosensor 110 capable of measuring an electrocardiogram. Here, the biosensor 110 may include a bioelectric electrode that may be attached to the skin to integrally measure an electrocardiogram, and a photoplethysmography sensor 110 that may indirectly measure a heart rate and an electrocardiogram through a change in blood flow.

The display 120 may output image data under the control of the processor 140. The display 120 may visually display various data processed by the one or more processors 140. The display 120 may output an image stored in the memory 130 under the control of the processor 140.

For example, the display 120 may output text, an image, a video, or the like related to the bio-age estimated by the processor 140. As a specific example, the display 120 may provide, in text, an image, and a video, the bio-age of the heart estimated by the processor 140, a difference between the bio-age and the actual age, and medical guidance corresponding to the age difference.

The display 120 may be implemented as an Liquid Crystal Display Panel (LCD), an Organic Light Emitting Diodes (OLED), or the like. In addition, the display 120 may be implemented as a flexible display 120, a transparent display 120, or the like in some cases.

Note that the type of the display 120 is merely exemplary, and is not necessarily limited to the specific type described above, and any medium that may visually represent image data may be applicable.

The memory 130 stores various data required by components of the device 100 for estimating a bio-age using a biomarker. For example, the data may include software (a program (e.g., middleware, an application), etc.) executed by the processor 140, a set of instructions, and input data and/or output data related to the instructions.

At this time, the input data and the output data may store basic information of the user (for example, gender, actual age, weight, and height), medical records (for example, past medical records indicating underlying diseases, existing diseases, and the like), electrocardiogram signals, preprocessing algorithms, artificial intelligence models, methods, and the like.

The processor 140 controls overall operation of the device 100 for estimating a bio-age using a biomarker. Specifically, the processor 140 is connected to the sensor 110, the display 120, and/or the memory 130, and may control operations overall by executing at least one instruction stored in the memory 130.

Specifically, the processor 140 may control, through instructions, the device 100 for estimating a bio-age using a biomarker to perform operations including receiving an electrocardiogram signal collected by the sensor 110 or received as input from an external device 100, preprocessing the electrocardiogram signal, selecting an artificial intelligence model to be used, extracting a biomarker from the electrocardiogram signal, applying a mathematical transformation to the biomarker, estimating the bio-age, and providing a health status corresponding to the estimated age.

The processor 140 may represent any type of processor 140 capable of executing firmware, middleware, software, and the like configured with instructions to cause the processor 140 to perform the described tasks.

Examples of the processor 140 include a central processing unit, a graphics processing unit, a digital signal processor, a display processor, a field programmable gate array, a microprocessor, an application-specific integrated circuit (for example, an artificial intelligence accelerator (ASIC)), and the like.

FIG. 2 is a block diagram of the processor 140 included in the device 100 for estimating a bio-age using a biomarker according to an embodiment.

Referring to FIG. 2, the processor 140 includes a receiving unit 141, a preprocessing unit 142, a first selection unit 143, an extraction unit 144, a validation unit 145, a second selection unit 146, a transformation unit 147, a modeling unit 148, and an output unit 149.

In the following embodiments, the receiving unit 141, the preprocessing unit 142, the first selection unit 143, the extraction unit 144, the validation unit 145, the second selection unit 146, the transformation unit 147, the modeling unit 148, and the output unit 149 may be implemented by using one or more physically separate devices, or may be implemented by one or more processors 140, or a combination of the one or more processors 140 and software, and specific operations may not be clearly separated from each other, which is different from the illustrated examples.

According to an embodiment, the receiving unit 141 may receive an electrocardiogram signal stored in the memory 130 or an electrocardiogram measured by the sensor 110. Here, the electrocardiogram signal may be a printed electrocardiogram image or a one-dimensional electrocardiogram signal of a single channel or multiple channels.

Herein, the electrocardiogram image may be an image including a graph recording electrical activity of the heart, and may be a two-dimensional image printed on paper or output on a digital screen from a signal output from an electrocardiogram device.

The one-dimensional electrocardiogram signal of a single or multiple channels may be understood as a signal continuously recorded of voltage changes over time corresponding to electrical activity of the heart, with each channel being measured at an independent electrode.

At this time, it is preferable that the electrocardiogram signal is a high-quality electrocardiogram signal that meets preset conditions. For example, it may be preferable that the electrocardiogram signal meets preset sampling rates, durations, qualities, sources, and noise filtering processing methods.

For example, the electrocardiogram signal may have a preset sampling rate, for example, a rate of 500 Hz. The electrocardiogram signal may have a duration of at least 2.5 seconds, preferably at least 10 seconds, such that sufficient measurements may be taken. The electrocardiogram signal may be accompanied by an indication of source of the data measurement equipment (e.g., wearable device, electrocardiogram monitor, 12-lead electrocardiogram machine, etc.). The electrocardiogram signal may be data that has been filtered to limit certain predetermined frequencies or that has been subjected to filtering in a particular order.

According to an embodiment, the preprocessing unit 142 may preprocess a received electrocardiogram signal.

The preprocessing unit 142 may perform noise removal processing on the received electrocardiogram signal. Specifically, when input data is an image printed on paper, the preprocessing unit 142 may remove logos, source indicators, and the like that are not necessary for feature extraction from the electrocardiogram waveform. When the input data is a digital signal, the preprocessing unit 142 may apply a band-pass filter or a notch filter to remove noise.

The preprocessing unit 142 may purify the received electrocardiogram signal. According to an embodiment, the preprocessing unit 142 may perform resizing or format conversion on the data size of the received electrocardiogram signal. For example, the preprocessing unit 142 may resize an image to meet a size specification that may be processed by a first model. Here, the image may be a printed image or data obtained by converting an electrocardiogram signal in a digital format into an image.

The preprocessing unit 142 may convert the received electrocardiogram signal. According to an embodiment, the preprocessing unit 142 may perform a standardization operation. Specifically, the preprocessing unit 142 may convert the electrocardiogram signal to have characteristics in various formats by converting a unit of the electrocardiogram signal to a voltage, performing resampling, and performing normalization. According to an embodiment, the preprocessing unit 142 may divide the received electrocardiogram signal into multiple segments. Here, the preprocessing unit 142 may divide the electrocardiogram signal including long-time-series data into segments continuously and non-overlappingly or overlappingly. According to an embodiment, the preprocessing unit 142 may divide the multi-channel electrocardiogram signal into segments having fixed intervals in a synchronized sampling manner. Here, the electrocardiogram signal may be divided into segments for each channel with respect to a same time period. According to an embodiment, the preprocessing unit 142 may generate segments in an asynchronized sampling manner. For example, the preprocessing unit 142 may apply an arbitrary time offset to segments for each channel. The preprocessing unit 142 may apply a random time shift to segments for each channel. The preprocessing unit 142 may cyclically shift time-series data of segments of each channel.

The first selection unit 143 may select a type of the first model to be used based on a signal representation scheme of the electrocardiogram signal.

For example, when the electrocardiogram signal is in an analog format, for example, when the input data is an image file on which a record of an electrocardiogram is printed, the first selection unit 143 may select a model configured with at least one of a vision transformer and a CNN as the first model.

As another example, when the electrocardiogram signal is in a digital format, for example, when the input data is a one-dimensional electrocardiogram signal of a single or multiple channels, the first selection unit 143 may select a model configured with at least one of an RNN and a CNN as the first model.

The extraction unit 144 may extract one or more biomarkers from the input electrocardiogram signal using the first model. The extraction unit 144 may extract one or more biomarkers from the input electrocardiogram signal using the first model.

Herein, the biomarker may refer to a feature and an indicator that may identify characteristics related to a health status of the heart that may be extracted from an electrocardiogram signal. That is, the biomarker may refer to a digital marker derived from an electrocardiogram signal (an electrocardiogram-derived digital marker). Specifically, the biomarker may include anatomical characteristics, physiological characteristics, and pathological characteristics related to the heart that may be estimated from the electrocardiogram signal.

The first model is a model trained with learning data to extract one or more biomarkers corresponding to an input electrocardiogram signal.

The training data may be configured such that an input value including an electrocardiogram signal and at least one characteristic of anatomical characteristics, physiological characteristics, and pathological characteristics corresponding to the electrocardiogram signal are labeled as a pair of ground truth values.

In one example, the anatomical characteristic is a characteristic related to cardiac hypertrophy, including at least one of the presence or absence of left atrial enlargement (LAE), the presence or absence of left ventricular hypertrophy (LVH), the presence or absence of right atrial enlargement, the presence or absence of right ventricular hypertrophy, a left ventricular mass index, and a left atrial volume index.

In one example, the physiological characteristic may include a characteristic related to systolic function, including the presence or absence of systolic or diastolic dysfunction of a left ventricle or a right ventricle, a left ventricular ejection fraction, myocardial strain, Brain Natriuretic Peptide (BNP), and Pro-brain Natriuretic Peptide (ProBNP).

In another example, the physiological characteristic may include a characteristic related to pulmonary arterial hypertension, including at least one of including pulmonary artery wedge pressure and the presence or absence of pulmonary hypertension. In another example, the physiological characteristic may include a characteristic related to cardiac ischemia, including at least one of a coronary artery calcium score, the presence or absence of coronary artery stenosis, the presence or absence of cardiac ischemia, and a troponin test result. In another example, the physiological characteristic may include a probability of death within a specified period of time.

In one example, the pathological characteristics may include characteristics related to valvular dysfunction, including at least one of the presence or absence and a severity score of stenosis or regurgitation of an aorta, a mitral valve, a lung, or a tricuspid valve.

The first model may be trained with augmented training data to improve the robustness of the model. Herein, the training data may be augmented by adding noise (e.g., Gaussian noise or random noise). The training data may be augmented by applying time distortion. For example, the training data may be randomly stretched or compressed along the time axis, thereby applying time distortion. The training data may also be augmented through random scaling. For example, the training data may be augmented by multiplying the magnitudes of the electrocardiogram signals within any range by random multiples.

It should be understood that in the second model, all of one or more biomarkers extracted by the extraction unit 144 may be used as independent variables in the second model, and a remaining part of one or more biomarkers extracted by the extraction unit 144, excluding a biomarker determined by the validation unit 145 to be inappropriate as described below, or a part of biomarkers selected by the second selection unit 146 may be used as independent variables in the second model.

For convenience of description, in embodiments where the second selection unit 146 is used, the biomarkers extracted by the extraction unit 144 are referred to as candidate biomarkers, and the biomarkers selected by the second selection unit 146 are referred to as one or more biomarkers for distinction in terminology. That is, it is intended that the markers finally used for the second model are used as one or more biomarkers defined in this specification.

The following is a description of the verification unit 145 and the second selection unit 146.

The verification unit 145 may verify the quality of the one or more biomarkers extracted by the extraction unit 144. Specifically, the verification unit 145 may verify the usefulness of the one or more biomarkers extracted by the extraction unit 144 by using a search data analysis technique. The verification unit 145 may verify whether values of the one or more biomarkers extracted by the extraction unit 144 have values within a predefined range. The verification unit 145 may also verify the validity of the format of whether the actual age of the patient and/or the health status related to the heart are indicated for each of the one or more biomarkers extracted by the extraction unit 144.

At this time, it is preferable that, among the one or more biomarkers extracted by the extraction unit 144, a low-quality biomarker that deviates from a preset level of usability or a value in a predefined interval or does not satisfy a data format is discarded. On the other hand, a high-quality biomarker may be stored in a structured format in the memory 130.

The second selection unit 146 may select some of one or more candidate biomarkers extracted by the extraction unit 144 as one or more biomarkers.

The second selection unit 146 may select some of the one or more candidate biomarkers as the one or more biomarkers based on a correlation between the extracted one or more candidate biomarkers and the actual age of the user. Here, it is assumed that the one or more candidate biomarkers include a plurality of biomarkers.

Specifically, the second selection unit 146 may select some of the candidate biomarkers showing a statistically significant relationship in the correlation test between the plurality of candidate biomarkers and the actual age as one or more biomarkers to be used in the second model. For example, the second selection unit 146 may perform a correlation test such as Pearson or Spearman on the relationship between the plurality of candidate biomarkers and the actual age, and select some of the candidate biomarkers showing a P-value lower than a predetermined significance level as one or more biomarkers to be used in the second model.

The second selection unit 146 may perform a correlation test on a relationship between the plurality of candidate biomarkers and the actual age, calculate an effect size of the correlation, and select some of the plurality of candidate biomarkers in an order of high clinical significance as one or more biomarkers to be used in the second model.

The second selection unit 146 may select one or more biomarkers to be used in the second model based on a result of performing multiple comparison correction on the plurality of candidate biomarkers. For example, the second selection unit 146 may select one or more biomarkers to be used in the second model by determining whether a corrected P-value obtained by applying Bonferroni Correction or False Discovery Rate (FDR) to a statistical test result between the plurality of candidate biomarkers and the actual age satisfies a significance level.

The second selection unit 146 may select some of the plurality of candidate biomarkers as one or more biomarkers to be used in the second model based on a result of visually analyzing a relationship between the plurality of candidate biomarkers and the actual age. For example, the second selection unit 146 may select one or more biomarkers to be used in the second model from the plurality of candidate biomarkers through a scatter plot.

The second selection unit 146 may select the remaining candidate biomarkers as one or more biomarkers to be used in the second model, excluding candidate biomarkers with low biological validity considering the relationship between the plurality of candidate biomarkers and the actual age.

The second selection unit 146 may divide the plurality of candidate biomarkers into groups and select a representative biomarker for each group as one or more biomarkers to be used in the second model. Specifically, the second selection unit 146 may cluster the plurality of candidate biomarkers into groups based on similarity, difference, biological/medical relevance among the candidate biomarkers, or a combination thereof. In this regard, the second selection unit 146 may select an optimal biomarker as a representative biomarker for each group, and select the representative biomarkers for each group as the one or more biomarkers to be used in the second model. Herein, the term "optimal" may refer to minimizing information redundancy among the remaining candidate biomarkers belonging to each group, best reflecting typical characteristics of the group, maximizing the prediction accuracy of the second model, or the like.

The calculation unit 147 may apply a mathematical transformation to the values of one or more biomarkers extracted by the extraction unit 144 or selected by the second selection unit 146. For example, the calculation unit 147 may perform a linearization transformation on the values of the one or more biomarkers, so that the correlation with the actual age may be linear and consistent. Accordingly, the calculation unit 147 may calculate the relationship between the one or more biomarkers and the actual age linearly, thereby improving the simplicity, interpretability, and calculation efficiency of the model.

The calculation unit 147 may calculate a linear correlation between a plurality of biomarkers and the actual age of the user while keeping the signs fixed. For example, when the correlation between the plurality of biomarkers and the actual age has a positive relationship, the calculation unit 147 may calculate a correlation with fixed signs while maintaining the direction of the relationship such that when the linearly transformed independent variable increases, the dependent variable also increases. For example, the calculation unit 147 may transform the plurality of biomarkers so that the order does not change according to the magnitude of the value through monotonic power transformation, and in this process, a linear relationship with age may be derived. Specifically, the monotonic power transformation may be implemented by a transformation function including a square root function, a cube root function, a logarithmic function, an inverse function, and/or a box-cox function. The transformation function may be optimized using a parameter.

Thus, the calculation unit 147 may reflect the biological characteristic that cardiac health consistently deteriorates with age through fixing the signs, and simplify the analysis of the correlation.

Herein, optimization may refer to maximizing a correlation coefficient or minimizing an error (for example, a mean squared error or a residual sum of squares) between a plurality of transformed biomarkers and an actual age. Alternatively, optimization may refer to a process in which a quality evaluation index (for example, Akaike Information Criterion (AIC) or Bayesian Information Criterion (BIC)) of a model is optimized. It is preferable that the optimization process be verified through statistical tests and visual plots to reveal its effectiveness.

The modeling unit 148 may model a second model using one or more biomarkers. Here, the second model is a learned regression model from which optimal regression coefficients are derived. The modeling unit 148 may model a regression model that estimates the bio-age of the user by using the extracted or selected one or more biomarkers as independent variables.

The modeling unit 148 may model a second model by adding a non-electrocardiogram biomarker as an independent variable. For example, the non-electrocardiogram biomarker may include a non-electrocardiogram biomarker extracted from vital signs, test results of a specimen, and the presence or absence of a concomitant disease in addition to information obtainable from an electrocardiogram.

In an example, the modeling unit 148 may model a second model configured as a constrained regression model. Here, the second model may include the following constraint conditions. The second model may include a first constraint condition that representative biomarkers exist in each group. The second model may include a second constraint condition that a magnitude of a correlation between one or more biomarkers and an actual age should fall within a preset sign or range. Here, the second model may include at least one of the first constraint condition and the second constraint condition as a constraint condition. Note that the first constraint condition is based on a precondition that a clustering process is performed on the plurality of candidate biomarkers by the second selection unit 146. That is, the second model may include the first constraint condition and the second constraint condition, or may include only the second constraint condition in some cases.

The modeling unit 148 may model the second model by using candidate biomarkers that satisfy the first constraint condition and/or the second constraint condition (hereinafter referred to as a constraint condition) as one or more biomarkers or limiting regression coefficients of biomarkers that do not satisfy the constraint condition to a certain range.

The output unit 149 may output a bio-age corresponding to the electrocardiogram signal by using the first model and the second model.

The output unit 149 may output a delta age, which is a difference between the bio-age and the actual age. In this case, the output unit 149 may output a grade corresponding to the delta age and a risk level of a corresponding disease. For example, when the delta age is in a difference range of 0 to 10 or more, a grade of 1 may be output; when the delta age is 11 to 20, a grade of 2 may be output; and when the delta age is more than 20, a grade of 3 may be output.

The output unit 149 may sequentially provide the biomarkers that contribute the most to estimating the bio-age. The output unit 149 may also output the significance of the contribution degrees of the biomarkers clinically.

The output unit 149 may output, in a visual format, a contribution degree of one or more biomarkers to estimating the bio-age. For example, the output unit 149 may visually output, using a coefficient plot, a waterfall plot, or a radar chart, a contribution degree of one or more biomarkers to outputting the bio-age.

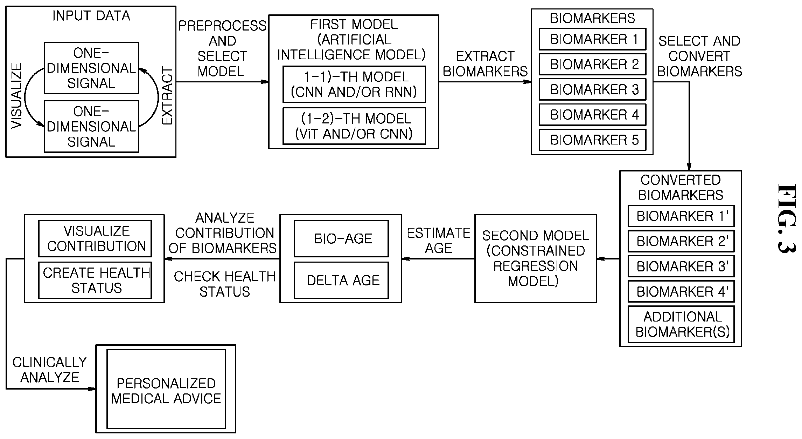

FIG. 3 is an exemplary diagram for describing an operation of the device 100 for estimating a bio-age using a biomarker according to an embodiment.

Referring to FIG. 3, first, the device 100 for estimating a bio-age using a biomarker according to an embodiment receives an electrocardiogram signal. Here, the electrocardiogram signal may be a one-dimensional electrocardiogram signal in a digital format or two-dimensional image data in which a one-dimensional electrocardiogram signal is recorded. Here, there is no limitation on the format, as the one-dimensional electrocardiogram signal may be extracted from the two-dimensional image, or the two-dimensional image may be interchangeable with an image showing the one-dimensional electrocardiogram signal.

Thereafter, the device 100 for estimating a bio-age using a biomarker according to an embodiment selects a type of first model suitable for processing based on a format of an electrocardiogram signal. For example, when the input electrocardiogram signal is a one-dimensional signal, a first model configured with an RNN that is strong in time-series feature extraction and a CNN that has learned a one-dimensional signal may be selected. As another example, when the input electrocardiogram signal is a two-dimensional signal, a first model configured with a vision transformer that is strong in image processing and a CNN may be selected. Meanwhile, it is preferable that the input electrocardiogram signal has been preprocessed so that the first model may process it.

Thereafter, the device 100 for estimating bio-age using a biomarker according to an embodiment extracts one or more candidate biomarkers from an electrocardiogram signal using the first model.

Herein, the candidate biomarker may refer to candidates of biomarkers to be used as modeling variables of a second model in the future. That is, a selected biomarker from the candidate biomarkers is finally used, and a biomarker that may be used is referred to as a candidate biomarker. It is preferable that the candidate biomarker is an index related to a health status of the heart, regardless of the age of the user, and an index related to the actual age is excluded.

Thereafter, the device 100 for estimating a bio-age using a biomarker according to an embodiment may select some of the one or more candidate biomarkers. Here, it is intended that the selected biomarkers are optimal biomarkers that are most helpful for determining the bio-age because they have a high correlation with the actual age or have no error.

Thereafter, the device 100 for estimating a bio-age using a biomarker according to an embodiment may construct a second model using the selected biomarkers. Here, the second model may be a constrained regression model in which the biomarkers satisfying the constraint condition serve as independent variables. Here, the biomarkers used as independent variables may be representative biomarkers selected from each group based on a classification criterion. Alternatively, they may refer to all the selected biomarkers without a classification process. Here, the biomarkers may refer to variables selected in a minimum number while maximizing the prediction performance of the second model.

Thereafter, the device 100 for estimating a bio-age using a biomarker according to an embodiment may estimate the bio-age, that is, the bio-age of the heart, using the second model, and estimate a delta age that is a difference between the bio-age and the actual age. The device 100 for estimating a bio-age using a biomarker according to an embodiment may not only provide the bio-age and the delta age on the display 120 but also visually provide a corresponding health status (risk level, disease severity) and a contribution of the biomarker according to the estimation of the bio-age.

Furthermore, the device 100 for estimating a bio-age using a biomarker according to an embodiment may provide personalized medical advice with clinical diagnosis based on at least one of the estimated bio-age, the delta age, the contribution of the biomarker, and the health status.

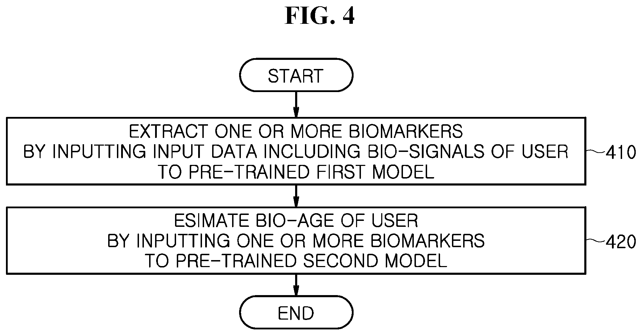

FIG. 4 is a flowchart for describing a method of estimating a bio-age according to an embodiment.

The method for estimating a bio-age according to an embodiment of FIG. 4 may be performed by the device 100 of FIG. 1.

First, a method for estimating a bio-age according to an embodiment includes extracting one or more biomarkers (410) by inputting input data including an electrocardiogram signal of a user to a first model.

Thereafter, the method of estimating a bio-age according to an embodiment includes estimating (420) a bio-age of the user on the basis of a second model modeled by using the one or more biomarkers.

Although the method is described in FIG. 4 by dividing it into a plurality of steps, at least some steps may be performed in a different order, combined with other steps to be performed together, omitted, divided into detailed steps, or may be performed with one or more steps not shown added thereto.

### [Industrial Applicability]

The disclosed embodiments are a device and method for estimating a bio-age using a biomarker, and may be used in the digital healthcare industry in that biomarkers are extracted from electrocardiogram signals using an artificial intelligence model, and a bio-age may be estimated through a regression model established based on the extracted biomarkers.

## Claims

1. A device for estimating a bio-age using a biomarker, comprising:
one or more processors; and
one or more memories storing instructions executed by the one or more processors,
wherein the one or more processors are configured to:
extract one or more biomarkers by inputting input data including biological signals of a user to a pre-trained first model; and
estimate a bio-age of the user by inputting the one or more biomarkers to a pre-trained second model.

2. The device of claim 1,
wherein the biological signal comprises an electrocardiogram signal, and
the bio-age comprises a cardiac bio-age.

3. The device of claim 1,
wherein the first model comprises an artificial intelligence model trained to output one or more corresponding biomarkers when receiving training data related to an electrocardiogram signal, and
the second model comprises a constrained regression model that models a part of the output data in the training process of the first model as independent variables, is trained to select regression coefficients and independent variables that minimize an error with ground truth values, and is trained to output a corresponding bio-age when receiving values of the output data as input.

4. The device of claim 3,
wherein the training data of the first model is configured such that an input value including an electrocardiogram signal; and at least one characteristic among anatomical characteristics, physiological characteristics, and pathological characteristics corresponding to the electrocardiogram signal are labeled as a ground-truth pair.

5. The device of claim 4,
wherein the anatomical characteristic is a characteristic related to cardiac hypertrophy, including at least one of the presence or absence of left atrial enlargement (LAE), the presence or absence of left ventricular hypertrophy (LVH), the presence or absence of right atrial enlargement, the presence or absence of right ventricular hypertrophy, a left ventricular mass index, and a left atrial volume index,
wherein the physiological characteristic comprises at least one of:
a characteristic related to systolic function, the characteristic comprising at least one of the presence or absence of systolic or diastolic dysfunction of a left ventricle or a right ventricle, a left ventricular ejection fraction, myocardial strain, brain natriuretic peptide (BNP), and pro-brain natriuretic peptide (ProBNP);
a characteristic related to pulmonary arterial hypertension, the characteristic comprising at least one of the presence or absence of pulmonary artery wedge pressure and the presence or absence of pulmonary hypertension;
a characteristic related to cardiac ischemia, the characteristic comprising at least one of a coronary artery calcium score, the presence or absence of coronary artery stenosis, the presence or absence of cardiac ischemia, and a troponin test result; and
a probability of death within a specified period of time, and
wherein the pathological characteristic is a characteristic related to valvular dysfunction, the characteristic comprising at least one of the presence or absence or a severity score of stenosis or regurgitation of an aorta, a mitral valve, a lung, or a tricuspid valve.

6. The device of claim 1,
wherein the one or more processors are configured to:
extract one or more candidate biomarkers by inputting an electrocardiogram signal of the user to the first model; and
extract the one or more biomarkers by selecting some of the one or more candidate biomarkers as the one or more biomarkers.

7. The device of claim 6,
wherein the one or more processors are configured to:
select some of the one or more candidate biomarkers as the one or more biomarkers based on at least one of a correlation between the one or more candidate biomarkers and an actual age of the user, an effect size of the correlation, and a biological plausibility of the one or more candidate biomarkers.

8. The device of claim 6,
wherein the one or more candidate biomarkers are a plurality, and
wherein the one or more processors are configured to:
cluster the one or more candidate biomarkers into respective groups based on a classification criterion,
select one or more representative biomarkers for each of the groups; and
estimate the bio-age by inputting the one or more representative biomarkers to the second model.

9. The device of claim 8,
wherein the classification criterion comprises at least one of similarity and biological or medical relevance among the one or more candidate biomarkers.

10. The device of claim 1,
wherein the one or more processors are configured to:
visually display, on a screen of a device in communication with the processor, the bio-age, together with at least one of: a contribution of the one or more biomarkers in determining the bio-age, a description regarding the contribution, a difference between the user's cardiac bio-age and actual age, a grade corresponding to the difference, a health status according to the grade, and guidance for improving the health status.

11. The device of claim 1,
wherein the one or more processors are configured to:
perform a linear transformation on the one or more biomarkers such that the one or more biomarkers have a linear correlation having a fixed sign with the user's actual age; and
calculate a correlation between the one or more biomarkers that have completed the linear transformation and the actual age.

12. The device of claim 8 or 11,
wherein the second model comprises a constrained regression model,
wherein a first constraint of the constrained regression model is that the one or more representative biomarkers exist in groups, and a second constraint of the constrained regression model is that the magnitude of the correlation is within a preset range.

13. The device of claim 1,
wherein the input data comprises at least one of a one-dimensional signal representing an electrocardiogram of the user in a time series and a two-dimensional signal representing the electrocardiogram as an image,
the first model comprises at least one of a vision transformer, a convolutional neural network, and a recurrent neural network, and
the one or more processors are configured to:
when a type of the input data is a one-dimensional signal, input the input data into a first-1 model composed of at least one of the convolutional neural network and the recurrent neural network, and
when a type of the input data is a two-dimensional signal, input the input data into a first-2 model composed of at least one of the vision transformer and the convolutional neural network.

14. A method performed by a device for estimating a bio-age using a biomarker, the device comprising one or more processors; and
one or more memories storing instructions executed by one or more processors,
the method comprising:
extracting one or more biomarkers by inputting input data including biological signals of a user to a pre-trained first model; and
estimating a bio-age of the user by inputting the one or more biomarkers to a pre-trained second model.

15. The method of claim 14,
wherein the biological signal comprises an electrocardiogram signal, and
the bio-age comprises a cardiac bio-age.

16. The method of claim 14,
wherein the first model comprises an artificial intelligence model trained to output one or more corresponding biomarkers when receiving training data related to an electrocardiogram signal, and
the second model comprises a constrained regression model that models a part of the output data in the training process of the first model as independent variables, is trained to select regression coefficients and independent variables that minimize an error with ground truth values, and is trained to output a corresponding bio-age when receiving values of the output data as input.

17. The method of claim 16,
wherein the training data of the first model is configured such that an input value including an electrocardiogram signal; and at least one characteristic among anatomical characteristics, physiological characteristics, and pathological characteristics corresponding to the electrocardiogram signal are labeled as a ground-truth pair.

18. The method of claim 17,
wherein the anatomical characteristic is a characteristic related to cardiac hypertrophy, the characteristic comprising at least one of the presence or absence of left atrial enlargement (LAE), the presence or absence of left ventricular hypertrophy (LVH), the presence or absence of right atrial enlargement, the presence or absence of right ventricular hypertrophy, a left ventricular mass index, and a left atrial volume index,
wherein the physiological characteristic comprises at least one of:
a characteristic related to systolic function, the characteristic comprising at least one of the presence or absence of systolic or diastolic dysfunction of a left ventricle or a right ventricle, a left ventricular ejection fraction, myocardial strain, brain natriuretic peptide (BNP), and pro-brain natriuretic peptide (ProBNP);
a characteristic related to pulmonary arterial hypertension, the characteristic comprising at least one of the presence or absence of pulmonary artery wedge pressure and the presence or absence of pulmonary hypertension;
a characteristic related to cardiac ischemia, the characteristic comprising at least one of a coronary artery calcium score, the presence or absence of coronary artery stenosis, the presence or absence of cardiac ischemia, and a troponin test result; and
a probability of death within a specified period of time, and
wherein the pathological characteristic is a characteristic related to valvular dysfunction, the characteristic comprising at least one of the presence or absence and a severity score of stenosis or regurgitation of an aorta, a mitral valve, a lung, or a tricuspid valve.

19. The method of claim 14,
wherein the step of extracting one or more biomarkers comprises:
extracting one or more candidate biomarkers by inputting an electrocardiogram signal of the user to the first model; and
extracting the one or more biomarkers by selecting some of the one or more candidate biomarkers as the one or more biomarkers.

20. The method of claim 19,
wherein the step of extracting one or more biomarkers comprises:
selecting some of the one or more candidate biomarkers as the one or more biomarkers based on at least one of a correlation between the one or more candidate biomarkers and an actual age of the user, an effect size of the correlation, and a biological plausibility of the one or more candidate biomarkers.

21. The method of claim 19,
wherein the one or more candidate biomarkers are a plurality, and
wherein the method comprises:
clustering the one or more candidate biomarkers into respective groups based on a classification criterion,
selecting one or more representative biomarkers for each of the groups; and
estimating the bio-age by inputting the one or more representative biomarkers to the second model.

22. The method of claim 21,
wherein the classification criterion comprises at least one of similarity and biological or medical relevance among the one or more candidate biomarkers.

23. The method of claim 14, comprising:
visually displaying, on a screen of the device, the bio-age, together with at least one of: a contribution of the one or more biomarkers in determining the bio-age, a description regarding the contribution, a difference between the user's cardiac bio-age and actual age, a grade corresponding to the difference, a health status according to the grade, and guidance for improving the health status.

24. The method of claim 14, further comprising:
performing a linear transformation on the one or more biomarkers such that the one or more biomarkers have a linear correlation having a fixed sign with the user's actual age; and
calculating a correlation between the one or more biomarkers that have completed the linear transformation and the actual age.

25. The method of claim 21 or 24,
wherein the second model comprises a constrained regression model,
wherein a first constraint of the constrained regression model is that the one or more representative biomarkers exist, and a second constraint of the constrained regression model is that the magnitude of the correlation is within a preset range.

26. The method of claim 14,
wherein the input data comprises at least one of a one-dimensional signal representing an electrocardiogram of the user in a time series and a two-dimensional signal representing the electrocardiogram as an image,
the first model comprises at least one of a vision transformer, a convolutional neural network, and a recurrent neural network, and
wherein the method comprises:
when a type of the input data is a one-dimensional signal, inputting the input data into a first-1 model composed of at least one of the convolutional neural network and the recurrent neural network, and
when a type of the input data is a two-dimensional signal, inputting the input data into a first-2 model composed of at least one of the vision transformer and the convolutional neural network.
